# EUROPEAN PATENT APPLICATION

(11) **EP 4 740 919 A1**
(43) Date of publication of application: **13.05.2026**
(21) Application number: 25213053.9
(22) Date of filing: 03.11.2025
(51) Int. Cl.: A61F 13/15

(54) **A METHOD AND A MACHINE FOR MANUFACTURING ABSORBENT SANITARY ARTICLES**

(30) Priority: 08.11.2024 IT 202400025158
(71) Applicant: Fameccanica.Data S.p.A., 66020 San Giovanni Teatino (CH) (IT)
(72) Inventor: SABLONE, Gabriele, I-66020 San Giovanni Teatino (Chieti) (IT); GIANSANTE, Antonio, I-66020 San Giovanni Teatino (Chieti) (IT); CUCCHIELLA, Devin, I-66020 San Giovanni Teatino (Chieti) (IT)
(74) Representative: Buzzi, Notaro & Antonielli d'Oulx S.p.A.

(57) **Abstract**

Method for manufacturing absorbent sanitary articles, wherein a first (12) and a second continuous band (14) advance parallel to each other in a machine direction (X), and a plurality of first chassis (20) and second chassis (22) oriented transversely to the continuous bands (12, 14), spaced apart in the machine direction (X) and alternately inverted are fixed to the first (12) and second continuous band (14), wherein the method comprises cutting and removing front portions (30, 34) of the chassis (20, 22) and corresponding portions (104, 106) of the continuous bands (12, 14) to form a first and a second array of product blanks (34, 36) each having a respective continuous band (12, 14) and rear portions (28, 32) of respective chassis (20, 22) fixed thereto.

## Description

### Field of the invention

The present invention relates to a method and a machine for manufacturing absorbent sanitary articles.

The invention was developed particularly for manufacturing reclosable absorbent sanitary articles, such as diapers, training pants, absorbent articles for incontinent adults, etc.

The invention was specifically developed for application to manufacturing processes called "Cross Direction".

### Prior art

A well-established technique for manufacturing reclosable absorbent sanitary articles consists of forming a continuous moving composite web in a machine direction comprising two continuous bands parallel to the machine direction and a plurality of chassis with opposite ends fixed to the continuous bands and extending orthogonally to the machine direction.

Sometimes the manufacturing process involves using a single continuous band parallel to the machine direction, to which end portions of chassis extending orthogonally to the machine direction are fixed.

This manufacturing technique called Cross Direction differs from the manufacturing technique called Machine Direction, which involves manufacturing absorbent sanitary articles while they advance with their longitudinal axes parallel to the machine direction.

Examples of methods for manufacturing absorbent sanitary articles according to the Cross Direction technique are described in documents EP-A-1013251, IT1379452, IT1410464 and IT1410465 by the same applicant.

For manufacturing reclosable absorbent sanitary articles, openable and reclosable fastening elements spaced apart in the machine direction are applied to one of the continuous waistbands.

EP-A-1523968 by the same applicant describes a fastening element for manufacturing absorbent sanitary articles wearable like underpants, comprising a non-woven web folded in a general omega-shaped configuration and including surface fastening elements.

Modern machines for manufacturing absorbent sanitary articles have extremely high production capacities, on the order of 800-1000 pieces/minute. To make such high production rates possible, materials must be advanced along the production line at very high speeds, on the order of 350-550 meters/min.

When the advancement speeds of materials along the production line are too high, numerous problems must be addressed and solved.

For example, if materials do not have sufficient time to adhere properly, defects may form at the joining points between various components, which can lead to loss of absorbency and compromise the structural integrity of the products.

At high speeds, materials are subject to greater stress and may involve higher risks of machine jams or stoppages. Moreover, as speed increases, lightweight and delicate materials are subject to higher risks of damage.

With excessively high speeds, materials may not be positioned correctly during the assembly process, leading to misalignment with consequent increase in product defects and waste.

Furthermore, increasing machine speeds can lead to premature wear and reduced service life of certain mechanical components, as well as increased maintenance costs.

### Object and summary of the invention

The object of the present invention is to provide a method for manufacturing absorbent sanitary articles that solves the problems of the prior art.

In particular, the present invention aims to reduce the advancement speed of materials along the production line without compromising the production capacity (throughput) of the machine.

According to the present invention, this object is achieved by a method having the features forming the subject of claim 1.

According to another aspect, the invention relates to a machine for manufacturing absorbent sanitary articles according to claim 7.

Preferred embodiments form the subject of the dependent claims.

The claims form an integral part of the teaching provided in relation to the invention.

### Brief description of the drawings

The present invention will now be described in detail with reference to the accompanying drawings, given by way of non-limiting example, in which:
- Figures 1-14 are schematic views illustrating different phases of a method for manufacturing absorbent sanitary articles according to the present invention, and
- Figure 15 is a schematic side view of a machine for manufacturing absorbent sanitary articles configured to implement the method illustrated in Figures 1-14.

### Detailed description

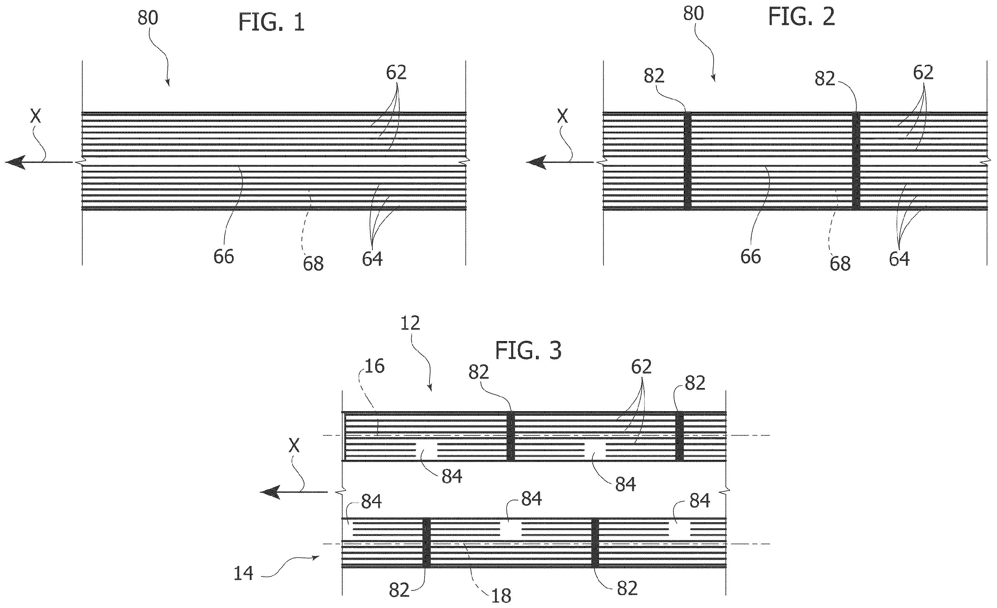

Figures 1-14 are schematic views illustrating a sequence of phases of a method for manufacturing absorbent sanitary articles according to the manufacturing technique called Cross Direction.

With reference to Figure 3, in a first phase the method involves forming a first and a second continuous band 12, 14 having respective longitudinal axes 16, 18 parallel to each other and advancing in a machine direction X parallel to the longitudinal axes 16, 18.

The "machine direction" in the context of manufacturing absorbent sanitary articles indicates the path along which raw materials advance and are transformed within production machines during the construction of absorbent sanitary articles.

The first and second continuous bands 12, 14 may be elastic bands stretchable in the longitudinal direction. The first and second continuous bands 12, 14 may comprise a plurality of first elastic threads 62 and a plurality of second elastic threads 64 parallel to each other and tensioned in the longitudinal direction, enclosed between respective layers of non-woven fabric and fixed thereto by respective patterns of fixing points formed by glue points or welding points.

With reference to Figures 1 and 2, in a possible embodiment, the method may involve forming a single continuous elastic band 80 comprising first and second elastic threads 62, 64 enclosed and fixed between two layers of non-woven fabric 66, 68. The single continuous elastic band 80 may be cut longitudinally to form the first and second continuous bands 12, 14 which, after longitudinal cutting, are spaced apart from each other in a transverse direction.

The method may involve cutting the first and second elastic threads 62, 64 in selected zones to form on the first and second continuous bands 12, 14 transverse non-elasticized areas 82 and central non-elasticized areas 84 whose function will become clear later.

With reference to Figure 3, the first and second continuous bands 12, 14 may be offset from each other in the machine direction X so that the transverse non-elasticized areas 82 of the first continuous band 12 are transversely aligned with the central non-elasticized areas 84 of the second continuous band 14 and the transverse non-elasticized areas 82 of the second continuous band 14 are transversely aligned with the central non-elasticized areas 84 of the first continuous band 12.

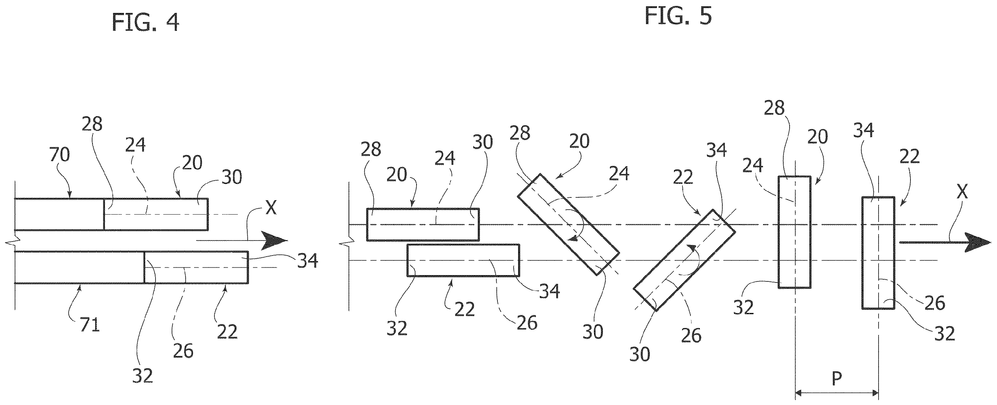

With reference to Figures 4 and 5, the method involves forming a plurality of first chassis 20 having respective chassis axes 24 and respective rear portions 28 and front portions 30, and a plurality of second chassis 22 having respective chassis axes 26 and respective rear portions 32 and front portions 34.

The first and second chassis 20, 22 may comprise respective absorbent cores enclosed between respective topsheets and backsheets, in a conventional manner in the field of manufacturing absorbent sanitary articles. The first and second chassis 20, 22 may also comprise accessory elements such as, for example, acquisition and distribution layers, elastic leg barriers (leg cuffs), and other components used for constructing absorbent sanitary articles.

With reference to Figure 4, the first and second chassis 20, 22 may be formed from respective continuous composite webs 70, 71 advancing along the machine direction X, which are cut transversely to form individual chassis 20, 22 separated from each other. In possible embodiments, the first and second chassis 20, 22 may be formed from a single continuous composite web 70.

With reference to Figure 5, the first and second chassis 20, 22 are alternately rotated by 90° in opposite directions and arranged with their respective chassis axes 24, 26 orthogonal to the machine direction X. The first and second chassis 20, 22 are also spaced apart from each other in the machine direction X and are arranged with a predetermined pitch P.

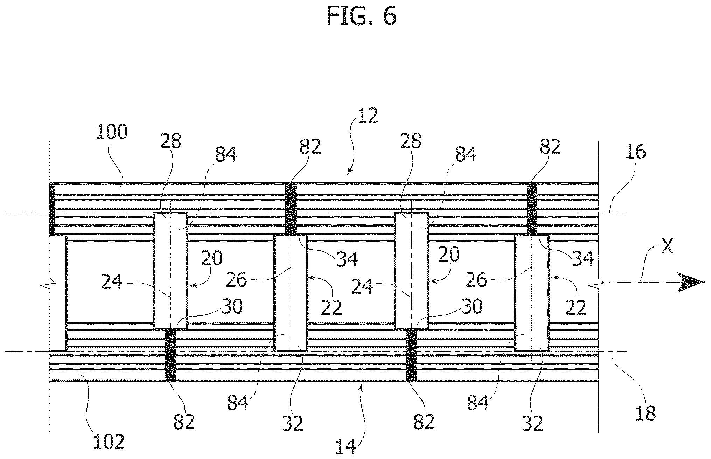

With reference to Figure 6, the first and second chassis 20, 22 are arranged with their respective chassis axes 24, 26 transverse to the longitudinal axes 16, 18 of the first and second continuous bands 12, 14. The first and second chassis 20, 22, spaced apart from each other in the machine direction X by a pitch P, are alternately inverted.

The first chassis 20 have their respective rear portions 28 overlapping the first continuous band 12 and their respective front portions 30 overlapping the second continuous band 14, and the second chassis 22 have their respective rear portions 32 overlapping the second continuous band 14 and their respective front portions 34 overlapping the first continuous band 12. The first chassis 20 and the second chassis 22 alternate with each other in the machine direction X.

The first chassis 20 and the second chassis 22 may be offset from each other in a direction transverse to the machine direction X so that the areas of the rear portions 28 of the first chassis 20 overlapping the first continuous band 12 are larger than the areas of the front portions 30 of the first chassis 20 overlapping the second continuous band 14, and the areas of the rear portions 32 of the second chassis 22 overlapping the second continuous band 14 are larger than the areas of the front portions 34 of the second chassis 22 overlapping the first continuous band 12.

In this phase, the rear portions 28 of the first chassis 20 are fixed to the first continuous band 12 and the front portions 30 of the first chassis 20 are fixed to the second continuous band 14. The rear portions 32 of the second chassis 22 are fixed to the second continuous band 14 and the front portions 34 of the second chassis 22 are fixed to the first continuous band 12. Such fixing may be carried out by glue applied intermittently on the first and second continuous bands 12, 14. The front portions 30, 34 of the first and second chassis 20, 22 may be fixed to the respective continuous bands 14, 12 by technical glue.

In possible application forms, the front portions 30, 34 and/or the rear portions 28, 32 of the first and second chassis 20, 22 are applied and fixed to the respective continuous bands 14, 12 so as to come into direct contact with the respective continuous bands 14, 12.

In possible application forms, the front portions 30, 34 and/or the rear portions 28, 32 of the first and second chassis 20, 22 are applied and fixed to the respective continuous bands 14, 12 indirectly, for example by interposing at least one layer of material between them.

The rear portions 28, 32 of the first and second chassis 20, 22 may be applied and fixed on respective central non-elasticized areas 84 of the first and second continuous bands 12, 14. The front portions 30, 34 of the first and second chassis 20, 22 may be applied and fixed on zones of the first and second continuous bands 12, 14 aligned with respective transverse non-elasticized areas 82.

The first and second continuous bands 12, 14 may have respective longitudinal edges 100, 102 folded onto respective inner surfaces and fixed to the respective inner surfaces along C-shaped fixing areas.

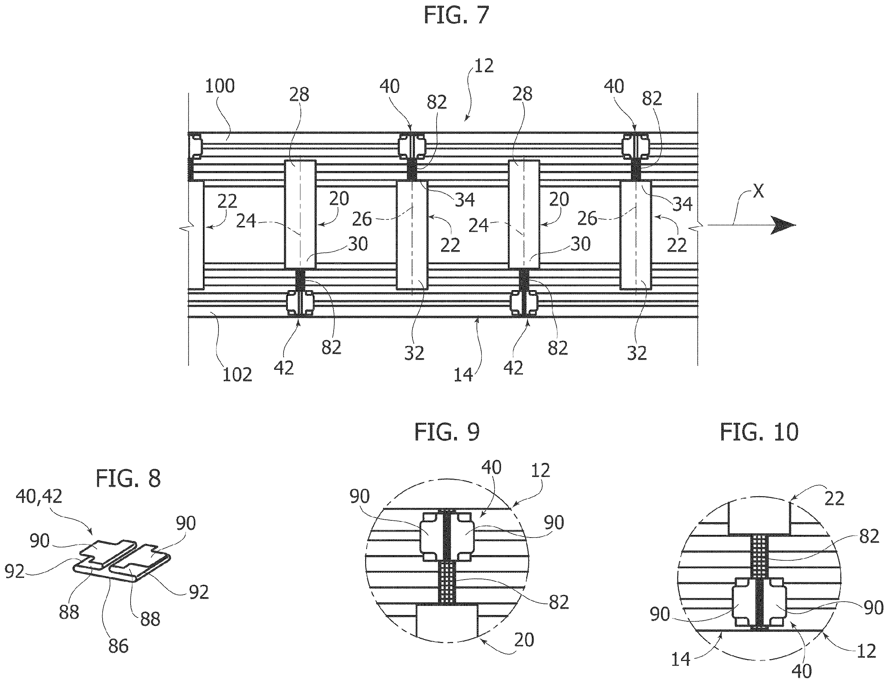

With reference to Figure 7, the method may involve forming a plurality of first fastening elements 40 and a plurality of second fastening elements 42.

With reference to Figure 8, each of the fastening elements 40, 42 may comprise a non-woven web folded in an omega shape and including a base 86, two intermediate flaps 88 connected to respective ends of the base 86 by respective first folds, two distal flaps 90 connected to respective ends of the respective intermediate flaps 88 by respective second folds, and two surface fastening elements 92 disposed on respective surfaces of the distal flaps 90 facing toward the respective intermediate flaps 88. The fastening elements 40, 42 may be formed as described in detail in Italian patent application No. 102023000026724 by the same applicant.

With reference to Figures 7, 9 and 10, the first fastening elements 40 are fixed to the first continuous band 12 in positions spaced apart from each other along the longitudinal axis 16 of the first continuous band 12 and the second fastening elements 42 are fixed to the second continuous band 14 in positions spaced apart from each other along the longitudinal axis 18 of the second continuous band 14.

The first fastening elements 40 are applied and fixed on respective transverse non-elasticized areas 82 of the first continuous band 12 and the second fastening elements 42 are applied and fixed on respective transverse non-elasticized areas 82 of the second continuous band 12.

The fixing of the first and second fastening elements 40, 42 to the continuous bands 12, 14 may be carried out by glue applied on the bases 86 of the fastening elements 40, 42 before their application to the respective continuous bands 12, 14.

The first fastening elements 40, fixed to the first continuous band 12, are transversely aligned with respective second chassis 22 and the second fastening elements 42, fixed to the second continuous band 14, are transversely aligned with respective first chassis 20.

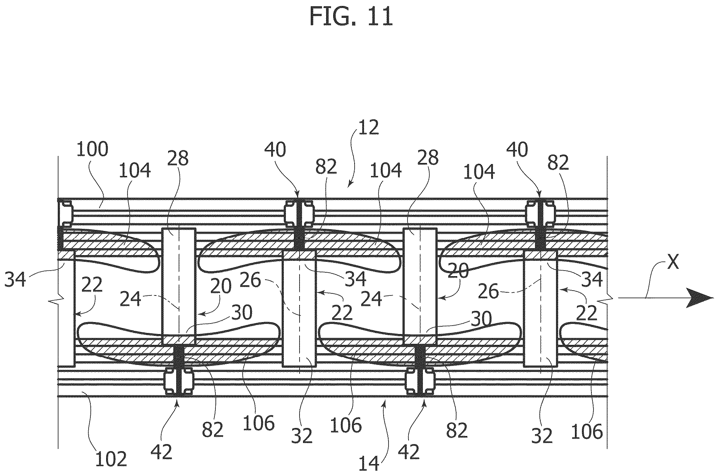

With reference to Figure 11, the method involves cutting portions 106 of the second continuous band 14 and portions 104 of the first continuous band 12 to form a first array of product blanks 36 including the first continuous band 12 and a plurality of first chassis 20 having their respective rear portions 28 fixed to the first continuous band 12, and a second array of product blanks 38 separated from the first array of product blanks 36 and including the second continuous band 14 and a plurality of second chassis 22 having their respective rear portions 32 fixed to the second continuous band 12.

In a possible embodiment, the method may involve cutting and removing front portions 30 of the first chassis 20 and corresponding portions 106 of the second continuous band 14, and front portions 34 of the second chassis 22 and corresponding portions 104 of the first continuous band 12.

These cuts separate the front portions 30, 34 from the remaining parts of the chassis 20, 22 and separate portions 104 and 106 of the continuous bands 12, 14 adjacent to respective inner edges. In Figure 11, the cut portions 104 and 106 are indicated by dashed lines. The cut portions 104 and 106 are removed and evacuated as waste. The cut portions 104 and 106 of the continuous bands 12, 14 may have the shape of lunettes, each of which has an arc extending above or below a rectilinear base. The cuts of the continuous bands 12, 14 may be shaped so that the absorbent sanitary articles being produced have waistbands with edges conformed to the legs of the users.

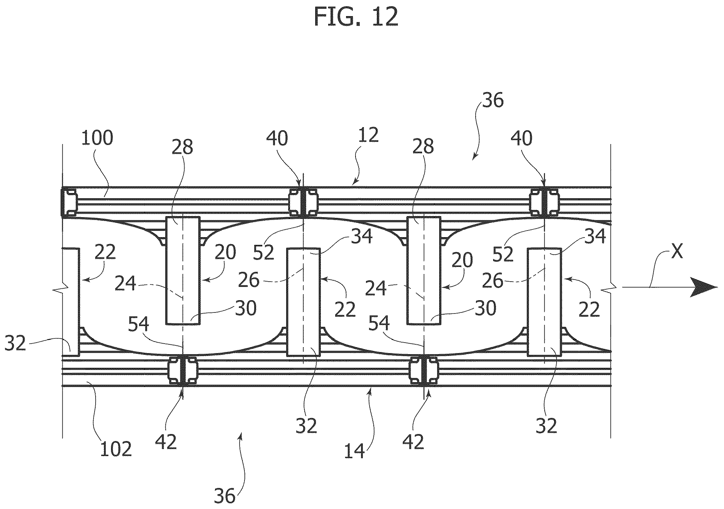

Figure 12 illustrates the shape of the structure after the cuts and removal of portions 104 and 106.

The cutting and removal phases of portions 104, 106 give rise to a first array of product blanks 36 including the first continuous band 12 and a plurality of first chassis 20 having their respective rear portions 28 fixed to the first continuous band 12, and a second array of product blanks 38, separated from the first array of product blanks 36, and including the second continuous band 14 and a plurality of second chassis 22 having their respective rear portions 32 fixed to the second continuous band 14.

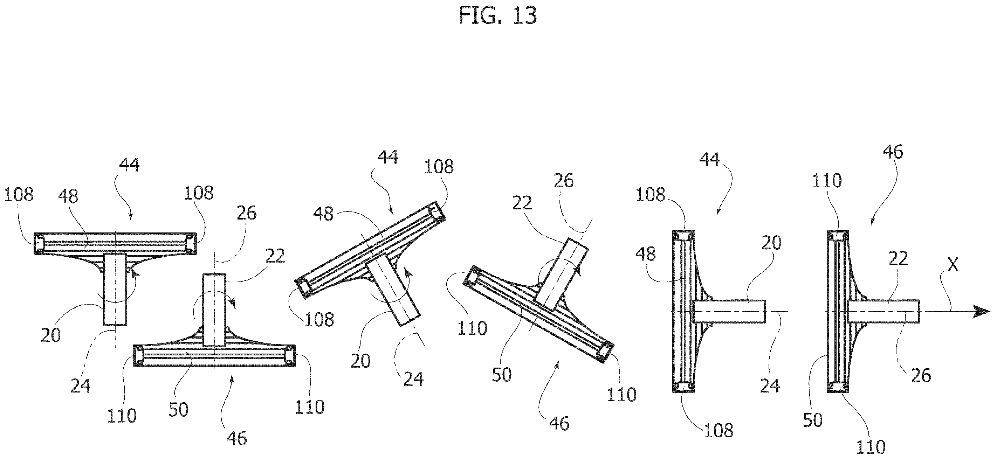

With reference to Figure 13, the method involves cutting the first continuous band 12 of the first array of product blanks 36 and the second continuous band 14 of the second array of product blanks 38 to form a plurality of first individual products 44 and a plurality of second individual products 46, each of which comprises a waistband 48, 50 and a respective chassis 20, 22.

With reference to Figure 12, the first continuous band 12 of the first array of product blanks 36 is cut along first cutting lines 52 extending along planes of symmetry of respective first fastening elements 40 and the second continuous band 14 of the second array of product blanks 38 is cut along second cutting lines 54 extending along planes of symmetry of respective second fastening elements 42. In this way, each of the first and second individual products 44, 46 has at each end of the respective waistband 48, 50 a side closure 108, 110 formed by half of a fastening element 40, 42.

With reference to Figure 13, the method may involve rotating the first and second individual products 44, 46 by 90° to orient the first and second individual products 44, 46 with their respective chassis axes 24, 26 parallel to the machine direction X. The first and second individual products 44, 46 may be alternately rotated in opposite directions from each other, so that after rotation the first and second individual products 44, 46 have the same orientation with respect to the machine direction X.

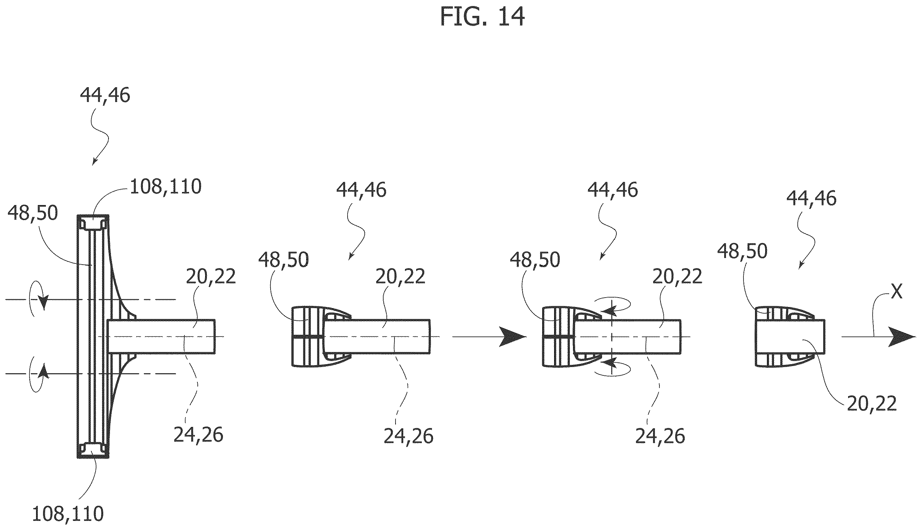

Thus, with reference to Figure 14, the method may involve folding the lateral portions of the waistbands 48, 50 onto their respective central portions around axes parallel to respective chassis axes 24, 26 and folding the chassis 20, 22 around axes transverse to respective chassis axes 24, 26.

The method may involve sending the first individual products 44 to a first folding device and sending the second individual products 46 to a second folding device.

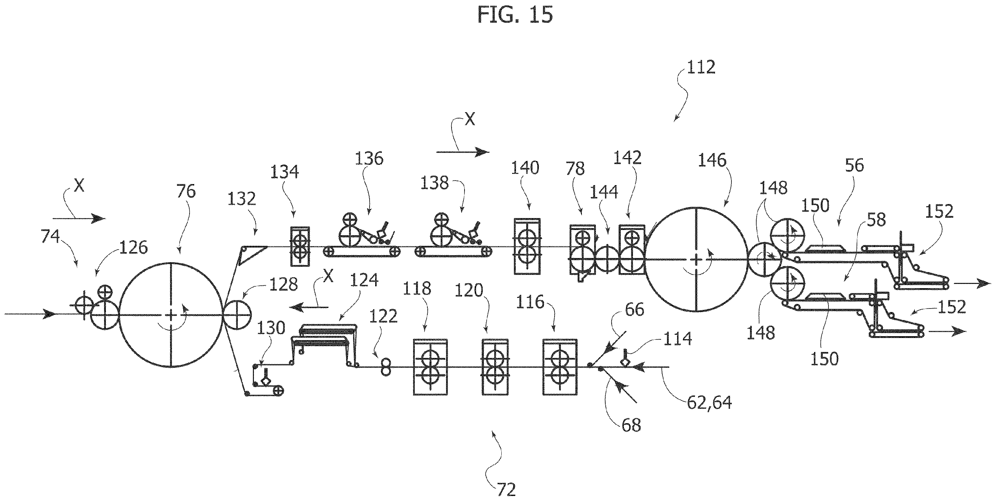

With reference to Figure 15, reference number 112 schematically indicates a machine for manufacturing absorbent sanitary articles configured to implement the previously described method.

The machine 112 comprises a band forming unit 72 configured to form a first and a second continuous band 12, 14 having respective longitudinal axes 16, 18 parallel to each other and advancing in a machine direction X parallel to said longitudinal axes 16, 18 (Figure 3).

The band forming unit 72 may comprise an elastic thread feeder for feeding elastic threads 62, 64 elastically tensioned in the longitudinal direction, a first glue applicator 114 for intermittently applying glue onto the elastic threads 62, 64, two feeders for feeding respective non-woven fabric webs 66, 68 from opposite sides of the elastic threads 62, 64 to form a single continuous elastic band 80.

The band forming unit 72 may comprise a first and a second elastic cutting device 116, 118 for cutting the first and second elastic threads 62, 64 to form transverse non-elasticized areas 82 and central non-elasticized areas 84. A pressing device 120 may be disposed between the first and second elastic cutting devices 116, 118.

The band forming unit 72 may comprise a longitudinal cutting device 122 for longitudinally cutting the single continuous elastic band 80 to form the first and second continuous bands 12, 14.

The band forming unit 72 may comprise a spreading device 124 for spacing apart the first and second continuous bands 12, 14 in a transverse direction. The spreading device 124 may also offset the first and second continuous bands 12, 14 from each other in the longitudinal direction.

The band forming unit 72 feeds the first and second continuous bands 12, 14 onto the outer surface of an anvil roller 128. Upstream of the anvil roller 128, a second glue applicator 130 may be disposed for intermittently applying glue onto the transverse non-elasticized areas 82 and central non-elasticized areas 84 of the continuous bands 12, 14.

The machine 112 comprises a chassis forming unit 74 configured to form a plurality of first chassis 20 and second chassis 22 having respective chassis axes 24, 26, respective front portions 28, 32 and respective rear portions 30, 34 (Figure 4).

The chassis forming unit 74 comprises a cutting unit 126 configured to transversely cut at least one continuous composite web 70, 71 advancing along the machine direction X to form individual first and second chassis 20, 22 separated from each other.

The machine 112 comprises a turn-and-repitch unit 76 configured to pick up the first and second chassis at the output of the cutting unit 126, to orient the first and second chassis 20, 22 with their respective chassis axes 24, 26 transverse to the machine direction X and to space apart the chassis 20, 22 in the machine direction X.

The turn-and-repitch unit 76 is configured to apply the first chassis 20 and second chassis 22 onto the first and second continuous bands 12, 14 on the anvil roller 128.

The turn-and-repitch unit 76 is configured to arrange the first chassis 20 and second chassis 22 alternately in the machine direction X and alternately oriented with their respective front portions 28, 32 and rear portions 30, 34 inverted, so that the first chassis 20 have their respective rear portions 28 overlapping the first continuous band 12 and their respective front portions 30 overlapping the second continuous band 14, and the second chassis 22 have their respective rear portions 32 overlapping the second continuous band 14 and their respective front portions 34 overlapping the first continuous band 12.

The turn-and-repitch unit 76 applies the first and second chassis onto the anvil roller 128 to fix rear portions 28 and front portions 30 of the first chassis 20 respectively to the first and second continuous bands 12, 14, and rear portions 32 and front portions 34 of the second chassis 22 respectively to the second and first continuous bands 14, 12.

The machine 112 may comprise an edge folding device 132 for folding the longitudinal edges 100, 102 of the first and second continuous bands 12, 14 and a pressing device 134 for stabilizing the folded longitudinal edges 100, 102.

The machine 112 may comprise a first and a second applicator device 136, 138 configured to apply and fix first fastening elements 40 to the first continuous band 12 and second fastening elements 42 to the second continuous band 14.

The machine 112 may comprise a pressing unit 140 to stabilize the fixing of the fastening elements 40, 42 to the first and second continuous bands 12, 14.

The machine 112 may comprise a shaped cutting unit 78 configured to cut and remove front portions 30 of the first chassis 20 and corresponding portions 106 of the second continuous band 14, and front portions 34 of the second chassis 22 and corresponding portions 104 of the first continuous band 12.

The cutting unit 78 forms a first array of product blanks 36 including the first continuous band 12 and a plurality of first chassis 20 having their respective rear portions 28 fixed to the first continuous band 12, and a second array of product blanks 38, separated from the first array of product blanks 36, and including the second continuous band 14 and a plurality of second chassis 22 (Figure 12).

The machine 112 may comprise a transverse cutting unit 142 for the transverse cutting of the first and second arrays of product blanks 36, 38 that gives rise to first and second absorbent sanitary articles 44, 46.

A transfer roller 144 may be disposed between the shaped cutting unit 78 and the transverse cutting unit 142.

The machine 112 may comprise a second turn-and-repitch unit 146 that performs the 90° rotation of the first and second absorbent sanitary articles 44, 46.

The machine 112 may comprise a first folding device 56 and a second folding device 58 and a series of transfer rollers 148 that send the first and second absorbent sanitary articles 44, 46 exiting the second turn-and-repitch unit 146 alternately to the first and second folding devices 56, 58.

Each of the folding devices 56, 58 may comprise a longitudinal folder 150 for longitudinally folding the waistbands 48, 50 of the first and second absorbent sanitary articles 44, 46 and a transverse folder 152 for transversely folding the chassis 20, 22 of the first and second absorbent sanitary articles 44, 46.

At the output of the machine 112, two flows of finished products ready for packaging can thus be obtained.

In summary, a machine 10 for manufacturing absorbent sanitary articles according to the present invention comprises:
- a band forming unit 72 configured to:
   ∘ feed a plurality of first elastic threads 62 and a plurality of second elastic threads 64 parallel to each other and tensioned in the longitudinal direction,
   ∘ enclose said first and second elastic threads 62, 64 between a first layer of non-woven fabric 66 and a second layer of non-woven fabric 68,
   ∘ fix said first and second elastic threads 62, 64 to the respective layers 66, 68 by a pattern of fixing points, so as to form a single continuous elastic band 80,
   ∘ cut said elastic threads 62, 64 in selected areas of the continuous elastic band 80 to form transverse non-elasticized areas 82 and central non-elasticized areas 84,
   o longitudinally cut said single continuous elastic band 80 to obtain a first continuous band 12 and a second continuous band 14 separated from each other in the transverse direction;
- a chassis forming unit 74 configured to form:
   o a plurality of first chassis 20 and a plurality of second chassis 22 having respectively chassis axes 24, 26, rear portions 28, 32 and front portions 30, 34;
- a turn-and-repitch unit 76 configured to:
   ∘ orient said first and second chassis 20, 22 with their respective chassis axes 24, 26 transverse to the machine direction X,
   ∘ arrange said chassis 20, 22 alternately and spaced apart from each other in the machine direction X and alternately inverted,
   ∘ overlapping:
      ▪ the rear portions 28 of the first chassis 20 to the first continuous band 12 and the front portions 30 to the second continuous band 14,
      ▪ the rear portions 32 of the second chassis 22 to the second continuous band 14 and the front portions 34 to the first continuous band 12,
   ∘ arrange the first and second chassis 20, 22 in transversely offset positions such that:
      ▪ the areas of the rear portions 28 overlapping the first continuous band 12 are larger than the corresponding front portions 30,
      ▪ the areas of the rear portions 32 overlapping the second continuous band 14 are larger than the corresponding front portions 34,
   o apply and fix:
      ▪ the rear portions 28 and front portions 30 of the first chassis 20 respectively to the first and second continuous bands 12, 14,
      ▪ the rear portions 32 and front portions 34 of the second chassis 22 respectively to the second and first continuous bands 14, 12;
- a shaped cutting unit 78 configured to:
   o cut portions 106 of the second continuous band 14 and portions 104 of the first continuous band 12 to form:
      ▪ a first array of product blanks 36 including the first continuous band 12 and a plurality of first chassis 20 fixed thereto,
      ▪ a second array of product blanks 38 separated from the first and including the second continuous band 14) and a plurality of second chassis 22 fixed thereto.

One of the main advantages of the method and machine according to the present invention is the possibility of halving the advancement speed of materials along the machine direction compared to a machine according to the prior art with the same production rate and with equal characteristics of the produced articles.

This allows, with equal production rate and equal characteristics of the produced articles, to increase the quality level of the produced articles and reduce waste due to defective articles.

The lower speed with equal production rate allows reducing wear of the mechanical components of the machine, with evident advantages in terms of fewer interventions and maintenance costs, and fewer machine stoppages for maintenance operations.

Naturally, while maintaining the principle of the invention, the construction details and embodiments may be widely varied from what has been described and illustrated without thereby departing from the scope of the invention as defined by the following claims.

### List of reference numbers

12 first continuous band
14 second continuous band
16 longitudinal axis of the first continuous band
18 longitudinal axis of the second continuous band
20 first chassis
22 second chassis
24 chassis axis of the first chassis
26 chassis axis of the second chassis
28 rear portions of the first chassis
30 front portions of the first chassis
32 rear portions of the second chassis
34 front portions of the second chassis
36 first array of product blanks
38 second array of product blanks
40 first fastening elements
42 second fastening elements
44 first individual products
46 second individual products
48 waistband of the first individual products
50 waistband of the second individual products
52 first cutting lines
54 second cutting lines
56 first folding device
58 second folding device
62 first elastic threads
64 second elastic threads
66 first layers of non-woven fabric
68 second layers of non-woven fabric
70 continuous composite web
71 second continuous composite web
72 band forming unit
74 chassis forming unit
76 turn-and-repitch unit
78 shaped cutting unit
80 single continuous elastic band
82 transverse non-elasticized areas
84 central non-elasticized areas
86 base of the fastening elements
88 intermediate flaps of the fastening elements
90 distal flaps of the fastening elements
92 surface fastening elements
100 longitudinal edges of the first continuous band
102 longitudinal edges of the second continuous band
104 cut portions of the first continuous band
106 cut portions of the second continuous band
108 side closure of the first individual products
110 side closure of the second individual products
112 machine for manufacturing absorbent sanitary
articles
114 first glue applicator
116 first elastic cutting device
118 second elastic cutting device
120 pressing device
122 longitudinal cutting device
124 spreading device
128 anvil roller
130 second glue applicator
132 edge folding device
134 pressing device for stabilizing folded edges
136 first applicator device
138 second applicator device
140 pressing unit
142 transverse cutting unit
144 transfer roller
146 second turn-and-repitch unit
148 series of transfer rollers
150 longitudinal folder
152 transverse folder

## Claims

1. A method for manufacturing absorbent sanitary articles (10), comprising:
- providing a plurality of first elastic threads (62) and a plurality of second elastic threads (64) parallel to each other and tensioned in the longitudinal direction,
- enclosing the first elastic threads (62) and the second elastic threads (64) between two layers of non-woven fabric (66, 68),
- fixing the first and second elastic threads (62, 64) to the first and second layers of non-woven fabric (66, 68) by a pattern of fixing points, so as to form a single continuous elastic band (80),
- cutting the first and second elastic threads (62, 64) of the single continuous elastic band (80) in selected areas to form transverse non-elasticized areas (82) and central non-elasticized areas (84),
- longitudinally cutting in the longitudinal direction said single continuous elastic band (80) to form a first and a second continuous band (12, 14) and spacing apart the first and second continuous bands (12, 14) in a transverse direction,
- providing a plurality of first chassis (20) having respective chassis axes (24) and respective rear portions (28) and front portions (30), and a plurality of second chassis (22) having respective chassis axes (26) and respective rear portions (32) and front portions (34),
- orienting said chassis (20, 22) with their respective chassis axes (24, 26) transverse to the machine direction (X), alternated and spaced apart from each other in the machine direction (X) and alternately inverted, so that the first chassis (20) have their respective rear portions (28) overlapping the first continuous band (12) and their respective front portions (30) overlapping the second continuous band (14), and the second chassis (22) have their respective rear portions (32) overlapping the second continuous band (14) and their respective front portions (34) overlapping the first continuous band (12),
- arranging the first chassis (20) and the second chassis (22) in positions offset from each other in a direction transverse to the machine direction (X) so that the areas of the first rear portions (28) overlapping the first continuous band (12) are larger than the areas of the first front portions (30) overlapping the second continuous band (14), and the areas of the second rear portions (32) overlapping the second continuous band (14) are larger than the areas of the second front portions (34) overlapping the first continuous band (12),
- applying and fixing rear portions (28) and front portions (30) of said first chassis (20) respectively to the first and second continuous bands (12, 14), and rear portions (32) and front portions (34) of said second chassis (22) respectively to the second and first continuous bands (14, 12), and
- cutting portions (106) of the second continuous band (14) and portions (104) of the first continuous band (12) to form a first array of product blanks (36) including the first continuous band (12) and a plurality of first chassis (20) having their respective rear portions (28) fixed to the first continuous band (12), and a second array of product blanks (38) separated from the first array of product blanks (36) and including the second continuous band (14) and a plurality of second chassis (22) having their respective rear portions (32) fixed to the second continuous band (12).

2. A method according to claim 1, comprising cutting and removing front portions (30) of said first chassis (20) and corresponding portions (106) of the second continuous band (14), and front portions (34) of said second chassis (22) and corresponding portions (104) of the first continuous band (12).

3. A method according to claim 1 or claim 2, comprising providing a plurality of first fastening elements (40) and a plurality of second fastening elements (42), fixing the first fastening elements (40) to the first continuous band (12) in positions spaced apart from each other along the longitudinal axis (16) of the first continuous band (12), and fixing the second fastening elements (42) to the second continuous band (14) in positions spaced apart from each other along the longitudinal axis (18) of the second continuous band (14).

4. A method according to claim 3, comprising fixing the first fastening elements (40) to the first continuous band (12) aligned with respective second chassis (22) and fixing the second fastening elements (42) to the second continuous band (14) aligned with respective first chassis (20).

5. A method according to claim 3 or claim 4, comprising cutting the first continuous band (12) along first cutting lines (52) extending along planes of symmetry of respective first fastening elements (40) and cutting the second continuous band (14) along second cutting lines (54) extending along planes of symmetry of respective second fastening elements (42) so that each of said first and second individual products (44, 46) has at each end of the respective waistband (48, 50) two side closures (108, 110) each of which is formed by half of a fastening element (40, 42).

6. A method according to any of the preceding claims, comprising cutting the first continuous band (12) of the first array of product blanks (36) and the second continuous band (14) of the second array of product blanks (38) to form a plurality of first individual products (44) and a plurality of second individual products (46), each of which comprises a waistband (48, 50) and a respective chassis (20, 22).

7. A machine (10) for manufacturing absorbent sanitary articles, comprising:
• a band forming unit (72) configured to:
∘ feed a plurality of first elastic threads (62) and a plurality of second elastic threads (64) parallel to each other and tensioned in the longitudinal direction,
∘ enclose said first and second elastic threads (62, 64) between a first layer of non-woven fabric (66) and a second layer of non-woven fabric (68),
∘ fix said first and second elastic threads (62, 64) to the respective layers (66, 68) by a pattern of fixing points, so as to form a single continuous elastic band (80),
∘ cut said elastic threads (62, 64) in selected areas of the continuous elastic band (80) to form transverse non-elasticized areas (82) and central non-elasticized areas (84),
∘ longitudinally cut said single continuous elastic band (80) to obtain a first continuous band (12) and a second continuous band (14) separated from each other in the transverse direction;
• a chassis forming unit (74) configured to form:
o a plurality of first chassis (20) and a plurality of second chassis (22) having respectively chassis axes (24, 26), rear portions (28, 32) and front portions (30, 34);
• a turn-and-repitch unit (76) configured to:
∘ orient said first and second chassis (20, 22) with their respective chassis axes (24, 26) transverse to the machine direction (X),
∘ arrange said chassis (20, 22) alternately and spaced apart from each other in the machine direction (X) and alternately inverted,
∘ overlapping:
▪ the rear portions (28) of the first chassis (20) to the first continuous band (12) and the front portions (30) to the second continuous band (14),
▪ the rear portions (32) of the second chassis (22) to the second continuous band (14) and the front portions (34) to the first continuous band (12),
∘ arrange the first and second chassis (20, 22) in transversely offset positions such that:
▪ the areas of the rear portions (28) overlapping the first continuous band (12) are larger than the corresponding front portions (30),
▪ the areas of the rear portions (32) overlapping the second continuous band (14) are larger than the corresponding front portions (34),
∘ apply and fix:
▪ the rear portions (28) and front portions (30) of the first chassis (20) respectively to the first and second continuous bands (12, 14),
▪ the rear portions (32) and front portions (34) of the second chassis (22) respectively to the second and first continuous bands (14, 12);
• a shaped cutting unit (78) configured to:
o cut portions (106) of the second continuous band (14) and portions (104) of the first continuous band (12) to form:
▪ a first array of product blanks (36) including the first continuous band (12) and a plurality of first chassis (20) fixed thereto,
▪ a second array of product blanks (38) separated from the first and including the second continuous band (14) and a plurality of second chassis (22) fixed thereto.
